# EUROPEAN PATENT APPLICATION

(11) **EP 3 449 817 A1**
(43) Date of publication of application: **06.03.2019**
(21) Application number: 18191536.4
(22) Date of filing: 29.08.2018
(51) Int. Cl.: A61B 5/00, A61B 8/00

(54) **ULTRASONIC PROBE AND PHOTOACOUSTIC APPARATUS INCLUDING THE ULTRASONIC PROBE**

(30) Priority: 30.08.2017 JP 2017165127
(71) Applicant: Canon Kabushiki Kaisha, Ohta-Ku Tokyo 1468501 (JP)
(72) Inventor: SUZUKI, Koichi, Ohta-ku, Tokyo 146-8501 (JP); ABE, Naoto, Ohta-ku, Tokyo 146-8501 (JP)
(74) Representative: Houle, Timothy James

(57) **Abstract**

An ultrasonic probe includes light irradiation means configured to irradiate a subject with light, acoustic wave receiving means configured to receive an acoustic wave generated when the subject is irradiated with the light and convert the acoustic wave into an electric signal, analog-to-digital conversion means configured to convert the electric signal into a digital signal, integration means configured to integrate a plurality of the digital signals obtained when the subject is irradiated with the light multiple times and output an integrated signal, and communication means configured to perform a communication of the integrated signal.

## Description

### BACKGROUND OF THE INVENTION

### Field of the Invention

The present invention relates to an ultrasonic probe and a photoacoustic apparatus including the ultrasonic probe.

### Description of the Related Art

Up to now, a photoacoustic apparatus that images a mode and a function inside a subject by using a photoacoustic effect has been researched. In the above-described related-art photoacoustic apparatus, when an instruction of a user is received, a controller inside the photoacoustic apparatus transmits a signal to a driving circuit of a light source, and the light source generates pulsed light. When the subject is irradiated with this pulsed light, a photoacoustic wave is generated. This photoacoustic wave is received by a probe and converted into an electric signal (photoacoustic signal). The controller performs signal processing such as image reconstruction on this photoacoustic signal and presents the obtained image to the user.

Japanese Patent Laid-Open No. 2017-006161 describes a photoacoustic imaging apparatus. According to Japanese Patent Laid-Open No. 2017-006161, a photoacoustic signal is obtained multiple times by a probe for each wavelength and transmitted to a main body side by a cable. Addition averaging of a plurality of photoacoustic signals is performed on the main body side to realize improvement in a signal/noise (S/N) ratio of the photoacoustic signals.

Meanwhile, in recent years, a wireless communication technology has been advanced, and improvement in a communication speed and miniaturization of a communication device have been progressed. PCT Japanese Translation Patent Publication No. 2002-530142 illustrates an example of an ultrasonic probe in which a data communication is performed between the main body and the probe.

In the photoacoustic apparatus described in Japanese Patent Laid-Open No. 2017-006161, as the number of ultrasonic wave receiving elements in the probe is increased for a higher image quality, the number of lines in the cable is increased. Thus, a cable diameter and a cable weight are increased. Accordingly, an issue has been found that it becomes difficult to handle the probe.

Even when the issue found in Japanese Patent Laid-Open No. 2017-006161 is dealt with on the basis of a wireless communication technology used in the ultrasonic probe described in PCT Japanese Translation Patent Publication No. 2002-530142, an issue unique to the photoacoustic apparatus may occur. That is, when the photoacoustic signal is to be obtained multiple times to attain a noise reduction effect, the communication data amount is significantly increased, and an issue occurs that a communication band is squeezed to decrease a frame rate.

The present invention has been made in view of the above-described issues and aims at providing a photoacoustic apparatus that can reduce the communication data amount when photoacoustic measurement is performed by obtaining the photoacoustic signal multiple times and suppress the decrease in the frame rate.

### SUMMARY OF THE INVENTION

The present invention in its first aspect provides an ultrasonic probe as specified in claims 1 to 7.

The present invention in its second aspect provides a photoacoustic apparatus as specified in claim 8.

Further features of the present invention will become apparent from the following description of exemplary embodiments with reference to the attached drawings.

### BRIEF DESCRIPTION OF THE DRAWINGS

Fig. 1 is a block schematic diagram for describing a photoacoustic probe according to an exemplary embodiment of the present invention.
Fig. 2 is an operation flow chart for describing the photoacoustic probe according to the exemplary embodiment of the present invention.
Fig. 3 is an operation flow chart for describing the photoacoustic probe according to the exemplary embodiment of the present invention.
Fig. 4 illustrates contents of a task memory for describing the photoacoustic probe according to the exemplary embodiment of the present invention.
Fig. 5 illustrates operation timings for describing the photoacoustic probe according to the exemplary embodiment of the present invention.

### DESCRIPTION OF THE EMBODIMENTS

Hereinafter, exemplary embodiments of the present invention will be described with reference to the drawings. It should be noted however that dimensions, materials, and shapes of constituent parts which will be described below, relative arrangements thereof, and the like are to be appropriately changed depending on a configuration of the apparatus to which the exemplary embodiment of the present invention is applied and various conditions, and the scope of this invention is not intended to be limited to the following descriptions. Furthermore, each of the embodiments of the present invention described below can be implemented solely or as a combination of a plurality of the embodiments or features thereof where necessary or where the combination of elements or features from individual embodiments in a single embodiment is beneficial.

The photoacoustic apparatus according to the present exemplary embodiment is an apparatus that uses a photoacoustic effect in which an acoustic wave generated inside the subject when the subject is irradiated with light (electromagnetic wave) is received, and characteristic information inside the subject is obtained. The characteristic information obtained at this time indicates a generation source distribution of the acoustic wave generated by the light irradiation, an initial sound pressure distribution inside the subject or an optical energy absorption density distribution or an absorption coefficient distribution derived from the initial sound pressure, a concentration distribution of a material constituting tissues, or the like. In addition, the characteristic information is typically image data, but numeric value information and the like may also be used as long as an internal state of the subject can be understood. The concentration distribution of the material includes, for example, an oxygen saturation distribution, an oxyhemoglobin/deoxyhemoglobin concentration distribution, or the like. Since the above-described characteristic information is also referred to as subject information, the photoacoustic apparatus according to the exemplary embodiment of the present invention can also be referred to as a subject information obtaining apparatus.

The acoustic wave mentioned in the exemplary embodiment of the present invention is typically an ultrasound wave and includes a sonic wave, an ultrasonic wave, and an elastic wave called an acoustic wave. The acoustic wave generated by the photoacoustic effect is also referred to as a photoacoustic wave or an optical ultrasonic wave in some cases. A probe of the subject information obtaining apparatus receives the acoustic wave generated inside the subject.

### First exemplary embodiment

According to the present exemplary embodiment, a probe unit (ultrasonic probe) of the photoacoustic apparatus obtains a photoacoustic signal multiple times and integrates those photoacoustic signals to be communicated to a main body part (signal processing unit).

### Photoacoustic apparatus

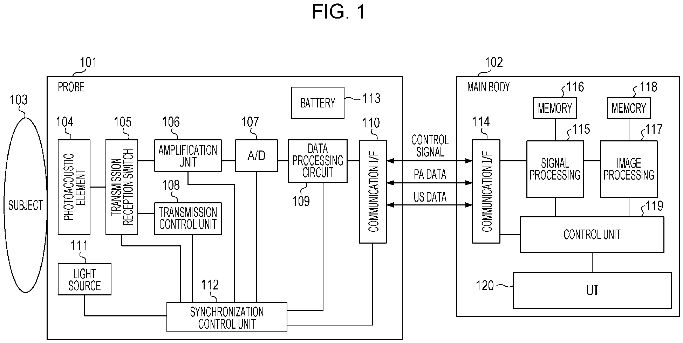

Fig. 1 is a block schematic diagram illustrating the photoacoustic apparatus according to the present exemplary embodiment. The photoacoustic apparatus according to the present exemplary embodiment includes an ultrasonic probe 101 and a signal processing unit 102. According to the present exemplary embodiment, the ultrasonic probe 101 is configured to obtain the photoacoustic signal mainly derived from the subject and perform a wireless data communication. The signal processing unit 102 is configured to mainly receive the data transmitted from the ultrasonic probe and obtain information of the subject. Hereinafter, each of the components will be described in detail.

### Ultrasonic probe

In Fig. 1, the ultrasonic probe 101 irradiates a subject 103 with light and also receives an acoustic wave generated in the subject 103 to convert the acoustic wave into an electric signal called a photoacoustic signal. The ultrasonic probe according to the present exemplary embodiment also includes a function of transmitting an ultrasonic wave to the subject 103 and receiving the ultrasonic wave that is reflected and returned from tissues inside the subject 103 to convert the ultrasonic wave into an electric signal called an ultrasonic signal. The ultrasonic probe 101 can also be referred to as a probe unit 101 of a photoacoustic apparatus. It should be noted that the light with which the subject is irradiated is typically pulsed light. A wireless data communication can be performed between the ultrasonic probe 101 and the signal processing unit 102, and it is possible to digitize a signal such as a control signal generated by the probe unit, the photoacoustic signal, or the ultrasonic signal to be transmitted to the main body part 102. According to the present exemplary embodiment, the digitized photoacoustic signal is referred to as photoacoustic signal data. Similarly, the digitized ultrasonic signal is referred to as ultrasonic signal data.

### Signal processing unit

The signal processing unit 102 in Fig. 1 can also be referred to as a main body part of the photoacoustic apparatus. The signal processing unit 102 receives the photoacoustic signal data and the ultrasonic signal data transmitted from the ultrasonic probe 101 by the wireless data communication and performs signal processing such as noise removal. In addition, imaging is carried out by performing image reconstruction processing of the obtained data, and the obtained image is presented to a user. According to the present exemplary embodiment, the signal processing unit includes a user interface (UI) 120 configured to input an instruction of the user.

### Subject

The subject 103 is a target where information is to be obtained by the photoacoustic apparatus and is a part of a body of a person being tested. Herein, a breast is taken as an example for the descriptions.

### Acoustic wave receiving unit

An acoustic wave receiving unit 104 receives a photoacoustic wave and an ultrasonic wave from the subject to be respectively converted into electric signals called a photoacoustic signal and an ultrasonic signal. The acoustic wave receiving unit 104 according to the present exemplary embodiment is also preferably configured to be able to transmit an ultrasonic wave to the subject 103.

According to the present exemplary embodiment, acoustic wave receiving units, in the acoustic wave receiving unit 104, having 100 channels or more are arranged in a one-dimensional array manner or a two-dimensional array manner in a plane where the ultrasonic probe is in contact with the subject. The acoustic wave receiving unit 104 is also constituted by including one or more acoustic elements. An example of an acoustic element is a configuration including a plurality of piezo elements or capacitance-type transducers. When an electric signal is applied to the acoustic element within the acoustic wave receiving unit 104, an ultrasonic wave is transmitted to the outside of the ultrasonic probe 101.

When the photoacoustic wave and the ultrasonic wave are received from the outside of the ultrasonic probe 101, electric signals are output. The acoustic wave receiving unit 104 may use the same element or mutually different elements at the time of the ultrasonic wave transmission and reception and the time of the photoacoustic wave reception.

### Switching circuit

A switching circuit 105 is configured to switch transmission and reception of the ultrasonic waves and is constituted by an analog switch, a multiplexer, a protecting diode, or the like. The switching circuit 105 is connected to a synchronization control unit 112 and switches connection between the corresponding acoustic wave receiving unit and a surrounding circuit respectively at the time of the ultrasonic wave transmission, the time of the ultrasonic wave reception, and the time of the photoacoustic wave reception. That is, when the ultrasonic wave is transmitted to the subject 103, a transmission control unit 108 and the acoustic wave receiving unit 104 are connected to each other, and the acoustic wave receiving unit 104 and the transmission control unit 108 are connected to each other. On the other hand, when the ultrasonic wave reflected from the subject 103 is received, an amplification unit 106 and the acoustic wave receiving unit 104 are connected to each other. In addition, when the photoacoustic wave from the subject is received too, the amplification unit 106 and the acoustic wave receiving unit 104 are connected to each other.

In a case where the different elements are used at the time of the ultrasonic wave transmission and reception and the time of the photoacoustic wave reception, the amplification unit 106 and the acoustic wave receiving unit 104 for the ultrasonic wave reception are connected to each other at the time of the ultrasonic wave reception, and the amplification unit 106 and the acoustic wave receiving unit 104 for the photoacoustic wave reception at the time of the photoacoustic wave reception. In addition, the number of channels of the transmission control unit is set to be low with respect to the number of channels of the acoustic wave receiving unit, and a plurality of elements may share a circuit.

### Pre-amplifier circuit

The amplification unit 106 is constituted by including a pre-amplifier circuit configured to amplify the photoacoustic signal and the ultrasonic signal output from the acoustic wave receiving unit 104. The amplification unit 106 includes a programmable gain amplifier and can dynamically change a gain by a setting from the outside. The amplification unit 106 is connected to the synchronization control unit 112 and can perform amplification processing at mutually different amplification factors (gains) at the time of the amplification of the photoacoustic signal and the time of the amplification of the ultrasonic signal on the basis of the instruction from the synchronization control unit 112. For example, in general, the photoacoustic signal is feebler (smaller) than the ultrasonic signal. When the photoacoustic signal is received, the gain is set to be relatively higher, and when the ultrasonic signal is received, the gain is set to be relatively lower. As an elapsed time since the ultrasonic wave transmission is longer, the gain is increased, and it is also possible to perform time gain control (TGC) processing for amplifying a feeble (small) signal from a deep part of the subject 103 at a still higher gain.

### Analog-to-digital conversion unit

An analog-to-digital conversion unit (A/D conversion unit) 107 digitizes the amplified signal into a digital signal. According to the present exemplary embodiment, the A/D conversion unit 107 is constituted by including an anti-aliasing filter for avoiding generation of repetitive noise, an A/D conversion circuit, and a memory that primarily saves the A/D converted photoacoustic signal data and ultrasonic signal data. The A/D conversion unit 107 is connected to the synchronization control unit 112 and performs sampling of the photoacoustic signal in synchronism with the control signal for the light irradiation to a light source 111. Sampling of the ultrasonic signal is also performed in synchronism with the control signal for the ultrasonic wave transmission from the transmission control unit 108.

### Transmission control unit

A transmission control unit 108 generates a pulse signal for driving the acoustic wave receiving unit 104. According to the exemplary embodiment, the transmission control unit 108 is constituted by including a high voltage pulsar circuit and a beam former circuit configured to generate driving pulses of the respective acoustic wave receiving units. The transmission control unit 108 controls phases of the signals to the respective acoustic wave receiving units on the basis of the setting from the synchronization control unit 112 and the control signal for the ultrasonic wave transmission and performs scanning of ultrasonic wave beam and transmit aperture control.

### Data processing circuit (integration unit)

A data processing circuit 109 integrates the photoacoustic signal data and the ultrasonic signal data digitized by the A/D conversion unit 107 to output an integration signal. In addition, division processing (addition averaging processing) for performing division the number of times corresponding to the number of times to perform the integration may be performed after the integration. A plurality of digital signals obtained when the subject is irradiated with the light multiple times are integrated to output the integration signal, and the integration signal is communicated by the communication unit, so that it is possible to decrease the communication data amount. As a result, the decrease in the frame rate can be suppressed.

The integration unit 109 is constituted by including, for example, FPGA, a micro controller, and the like. That is, the photoacoustic signal is received multiple times, and the photoacoustic signals are integrated to reduce random noise. The same location of the subject 103 is imaged N times, and addition averaging of the N photoacoustic signals is performed. In a case where the noise is the random noise, an advantage is attained that the size is set to be 1/√N. Since the size of the photoacoustic signal is not changed even after the addition averaging is performed, the signal to noise ratio can be multiplied by √N. Furthermore, the data processing circuit 109 is connected to the synchronization control unit 112 and converts the photoacoustic signal data and the ultrasonic signal data into a format appropriate to the communication to the main body. Specifically, the data compression processing, the noise removal processing, or the like is performed. At this time, different processing can be applied to the ultrasonic signal data and the photoacoustic signal data. For example, application of filter processing having different pass bands may be performed in accordance with frequencies of the respective signals at the time of the photoacoustic signal reception and the time of the ultrasonic signal reception. The data processing circuit also converts the ultrasonic signal data into a predetermined format, and additional information is added. The additional information includes an ID of the probe and information for distinguishing the photoacoustic signal data and the ultrasonic signal data from each other. In addition, information for distinguishing types of data for the photoacoustic signal data and the ultrasonic signal from each other is included. For example, in a case where the photoacoustic signal data with respect to light having a plurality of wavelengths is obtained at the time of the obtainment of the photoacoustic signal, information for identifying the wavelength is included as the additional information. In a case where data for B mode and data for Doppler are obtained at the time of the obtainment of the ultrasonic signal data, information for identifying the B mode and Doppler is included as the additional information. It should be noted that the integration unit may also be configured to be able to change the number of times to perform the integration of the electric signals.

### Communication unit

A communication unit (communication interface) 110 of the probe wirelessly transmits the ultrasonic signal data and the photoacoustic signal data obtained by the probe unit 101 to the main body part 102 and transmits and receives a control command from the main body part 102. The communication unit according to the present exemplary embodiment is basically a wireless communication interface but may separately include an interface that can transmit and receive data in a wired manner.

A wireless communication protocol such as Wi-Fi, Bluetooth (registered trademark), 2G, 3G, or 4G can be used as the communication protocol. The communication interface 110 is constituted by an input buffer, a communication module, and an antenna. The communication module is constituted by a baseband unit and an RF unit and performs addition of header information to the data, error correction, and packetization on the basis of the wireless communication protocol. Spectrum spread, modulation, or demodulation processing or the like is performed in accordance with the wireless communication protocol. In addition, the communication module performs retransmission control or the like at the time of a communication error.

The input buffer is a buffer memory that primarily stores the photoacoustic signal data and the ultrasonic signal data from the data processing circuit 109. The buffer memory is based on first in first out (FIFO). Data pieces output from the data processing circuit 109 are sequentially input on the basis of FIFO, and the data pieces input earlier are read out to a wireless communication module in sequence. The buffer memory is constituted by a dual port memory and can perform data write from the data processing circuit 109 and data read from the wireless communication module in parallel. In addition, it is possible to regularly monitor an internal state of the buffer memory from the synchronization control unit 112.

Contents of the control command transmitted from the main body part 102 to the probe unit 101 include imaging start, interruption, temporary pause, status notification, and the like. A photoacoustic signal obtaining function and parameters at the time of the obtainment of the ultrasonic signal are also included in the control command. The parameters include the presence or absence of the obtainment of each of the signals, an obtainment frequency, a type of a wavelength of the light source, an operation mode of the ultrasonic wave, a type of transmission beam forming, data compression rates of the respective signals, and the like.

It should be noted that the communication unit is preferably configured to be able to perform a wired communication according to the present exemplary embodiment. The frame rate of the wirelessly communicated electric signal is lower than the frame rate of the electric signal where the wired communication is performed.

In addition, the wirelessly communicated electric signal may be a signal to which the plurality of electric signals obtained during a certain period of time are added or an electric signal after the addition averaging which is obtained by adding and dividing by the number of signals. In this manner, when the electric signal after the addition or the addition averaging is used, it is possible to reduce the data amount of the wireless communication.

With regard to the timing of the wireless communication, in a case where the light is emitted from the light source multiple times, it is possible to perform the wireless communication during the light emission.

### Light source

The light source 111 emits pulsed light. According to the present exemplary embodiment, the light source is constituted by including, for example, a semiconductor laser device and a driving circuit. The light source 111 includes an external trigger signal and is configured to be able to control a timing of the light emission from the synchronization control unit 112. The light source 111 also includes a light amount setting signal and can set the light amount from the synchronization control unit 112. In a case where a set light amount is high, the driving circuit inside the light source increases a current value to the semiconductor laser device. On the other hand, in a case where the set light amount is low, the driving circuit decreases the current value to the semiconductor laser device. In addition, a plurality of semiconductor laser devices having different oscillation wavelengths may be provided, and light having different wavelengths may be emitted in accordance with the setting from the synchronization control unit 112.

It should be noted that a switching unit configured to determine the light emitting timing by the light source may be included in the probe unit.

A configuration may be adopted in which, when the data amount of the wirelessly communicated electric signal becomes higher than or equal to a predetermined value, a repetition frequency of the light emitted from the light source is set be decreased.

The light source may be constituted by including a plurality of semiconductor light emitting elements arranged in an array manner and may also be constituted by including a solid-state laser such as titan sapphire laser, Yag laser, or alexandrite laser. Examples of the semiconductor light emitting element include a light emitting diode (LED), semiconductor laser (LD), and the like.

### Synchronization control unit

The synchronization control unit 112 communicates with the respective units in the probe unit 101 and performs the synchronization control. The synchronization control unit 112 is constituted by a microcomputer and software. According to the present exemplary embodiment, control for shifting the wireless communication and the timing of the light emission of the laser is performed. The synchronization control unit can control the timing of the light emission by the light source and the timing of the wireless communication by the communication unit. For example, the synchronization control unit controls the timing of the light emission by the light source and the timing of the wireless communication by the communication unit such that the time of the light emission and the time of the wireless communication are not overlapped with each other. It should be noted that the synchronization control unit according to the present exemplary embodiment can perform the control such that the wireless communication is performed after the light emission by the light source. The synchronization control unit according to the present exemplary embodiment can also perform the control such that the light emission by the light source is performed after the wireless communication by the communication unit. A detail of the control contents will be described below. Power supply unit

A battery 113 supplies power to the respective units in the probe unit 101. A small-sized lithium ion battery or the like is used as the battery 113. The probe unit 101 includes a terminal for charging the battery 113 and can charge the battery by being connected to the main body part 102. The connection may be performed by a method of connecting a charging cable or a method of causing a charging terminal of the probe unit 101 to contact with a charging terminal part of the main body part 102. The remaining amount of the battery 113 is monitored by the microcomputer in the synchronization control unit 112, and a warming is displayed in a case where the remaining amount is becoming low. An LED or a small-sized liquid crystal display unit is installed on the probe unit 101, and the battery remaining amount and the warning may be displayed. In addition, the main body part 102 may be notified of a status via the wireless interface 110 in a stage where the battery remaining amount is becoming low, and the warning may be displayed on the user interface 120 on the main body part 102.

### Communication unit of the main body

A communication interface 114 wirelessly receives the ultrasonic signal data and the photoacoustic signal data obtained by the probe unit 101 in the main body part 102 and also transmits and receives the control to and from the probe unit 101. The communication interface 114 on the main body side is provided with the wireless communication function similar to that of the communication interface 110 on the probe side. The received ultrasonic signal data and the received photoacoustic signal data are transmitted to a signal processing circuit (digital circuit) 115.

The signal processing circuit 115 applies the signal processing to the ultrasonic signal data and the photoacoustic data. A field programmable gate array (FPGA), a digital signal processer (DSP), or the like is implemented as the signal processing circuit 115. The types of the ultrasonic signal data and the photoacoustic signal data are distinguished from each other on the basis of the information added by the data processing circuit 109, and different signal processes are respectively applied to the data. For example, phasing addition, logarithmic amplification, envelope detection processing, harmonic imaging processing, or the like is performed with respect to B mode data of the ultrasonic signal. Frequency analysis, HPF processing, or the like is performed with respect to Doppler data of the ultrasonic signal to generate data indicating a speed of bloodstream at an observation position. Response correction processing of the acoustic wave receiving unit, noise removal processing, bandpass filter processing, or the like is performed with respect to the photoacoustic signal data. When these processes are performed, the parameters of the signal processing may be changed in accordance with probe ID information included in the additional information. For example, at the time of the response correction processing of the acoustic wave receiving unit, the type of the acoustic wave receiving unit may be identified from the probe ID information to select a different impulse response waveform. At the time of the bandpass filter processing, a reception frequency band of the acoustic wave receiving unit may also be identified from the probe ID information to select a filter corresponding to the reception frequency band. In addition, the wavelength of the light source 111 the photoacoustic signal data when is obtained from the additional information is identified, such that photoacoustic signal data of a different wavelength is not to be mixed.

### Storage unit

A storage unit (memory) 116 saves the ultrasonic signal data and the photoacoustic signal data on which the signal processing has been performed by the signal processing circuit 115. The respective pieces of data are saved in different areas in accordance with the types by the signal processing circuit 115.

An image processing circuit 117 is a circuit configured to read out the data saved in the memory 116 and perform imaging in accordance with the type of the data and is constituted by a processor (graphics processing unit (GPU)) dedicated to the image processing. The B mode data of each of scanning lines are combined with respect to the ultrasonic signal data to generate a B mode image. The image reconstruction processing is performed on the photoacoustic signal data to generate an initial sound pressure distribution and absorption coefficient distribution image. Universal back projection (UBP), model-based reconstruction processing, or the like has been proposed as an algorithm of the image reconstruction processing. In addition, an oxygen saturation distribution is calculated by using the photoacoustic data of a plurality of wavelengths.

A memory 118 saves data imaged by the image processing circuit 117. An HDD, an SSD, or the like is used as the memory.

It should be noted that a configuration may also be adopted in which control is performed such that the repetition frequency of the pulsed light emitted from the light irradiation unit is decreased in a case where the data amount saved in the memory unit is higher than or equal to a predetermined value.

A control unit 119 is a processor connected to the respective units on the main body side and controls the entirety. The control unit 119 is constituted by a CPU and software running on the CPU. The control unit 119 receives the imaging instruction and the imaging parameters input from the user via the user interface 120 and outputs a command to the probe unit 101 via the communication interface 114. The probe unit 101 obtains the ultrasonic signal data and the photoacoustic signal data on the basis of the contents of the command received via the communication interface 110 and transmits the obtained data to the main body part 102 via the communication interface 110. In the main body part 102, the ultrasonic signal data and the photoacoustic signal data are received via the communication interface 114, and a diagnostic image is generated by the signal processing circuit 115 and the image processing circuit 117. The diagnostic image is output to the user interface 120 via the control unit 119 to be presented to the user.

The user interface 120 accepts the instruction input to the photoacoustic apparatus from the user and also outputs the diagnostic image to the user. Specifically, the user interface 120 is constituted by a keyboard, a mouse, a display, or the like. Operation flow of the signal processing unit

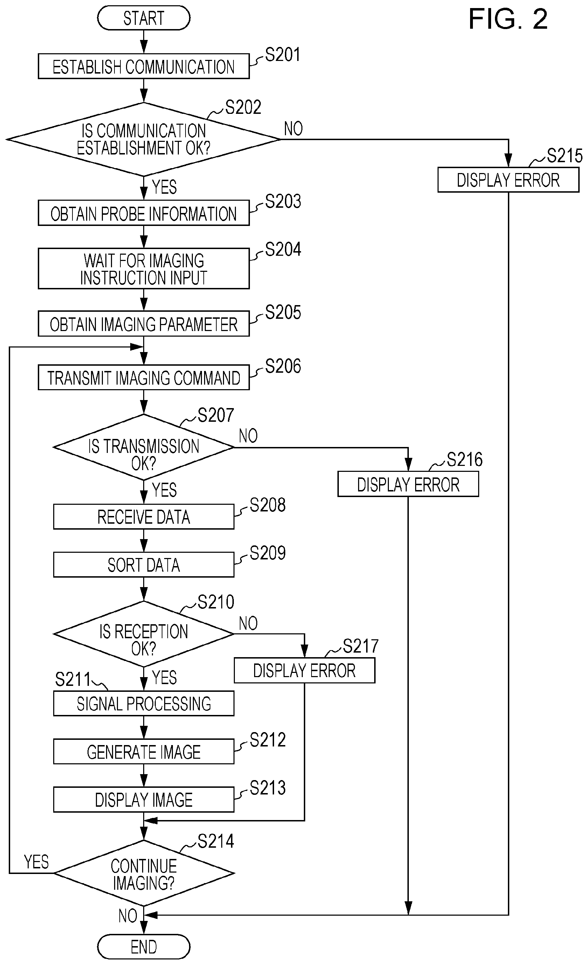

Next, a flow of an operation performed inside the main body part 102 will be described. Fig. 2 is a flow chart illustrating operations of the respective units in the main body part 102.

### Step S201

In step S201, the control unit 119 transmits an instruction to the communication interface 114 to transmit a communication command. A probe having a probe ID registered by the user is searched for, and a communication is regarded as established when a response is issued. In a case where responses are issued from a plurality of probes, a list of communicable probes is displayed on the user interface 120 to be selected by the user.

### Step S202

Next, in step S202, it is determined whether or not the communication with the probe unit 101 is established. In a case where the communication is not established, it is determined that a usable probe does not exist in the surrounding or the battery 113 runs out, and the flow proceeds to step S215. In a case where the communication with the probe unit 101 is established, the flow proceeds to step S203.

### Step S203

In step S203, the probe unit 101 is requested for information of the probe. Specifically, information such as a type of the wavelength of the light source 111, a maximum frequency at which the light can be emitted, the number of elements of the acoustic wave receiving unit 104, a bandwidth, a center frequency, the number of bits of the A/D converter, the remaining amount of the battery 113, and a communication speed of the communication interface 110 is obtained. The obtained information is saved in the memory of the control unit 119.

### Step S204

In step S204, the flow waits for the user to input the imaging parameters and the imaging instruction via the user interface 120. When the input is completed, the flow proceeds to step S205.

### Step S205

In step S205, the imaging parameters are saved in the memory in the control unit 119. The imaging parameters include a type of the diagnostic image to be obtained, a depth of an imaging target, an imaging range of the ultrasonic wave, a repetition frequency of the ultrasonic wave, the presence or absence of bloodstream measurement based on Doppler, an irradiation cycle by the pulsed light, the number of times to perform the irradiation, the wavelength, and the like.

### Step S206

In step S206, the control unit 119 transmits an instruction to the communication interface 114 to transmit the imaging command to the probe 101. The imaging command includes parameters used for the ultrasonic signal obtainment and parameters used for the photoacoustic signal obtainment. Specifically, the parameters used for the ultrasonic signal obtainment include the number of ultrasonic wave beams, the repetition frequency, a beam scanning method, a focus point, a type of a gain table based on time gain control (TGC), and the like. The parameters used for the photoacoustic signal obtainment include the repetition frequency of the light source, the number of times to perform the light irradiation, a light irradiation intensity, a type of the wavelength, the gain at the time of the reception, the number of times to perform the integration of the reception signals, and the like. The parameters also include temporal parameters of the ultrasonic signal obtainment and the photoacoustic signal obtainment. For example, the parameters include information indicating whether the ultrasonic signal obtainment and the photoacoustic signal obtainment are alternately performed or the ultrasonic signal obtainment is continuously performed N times and thereafter the photoacoustic signal obtainment is continuously performed M times or the like. The control unit 119 generates an imaging command by finding out these pieces of information used for the ultrasonic signal obtainment and the photoacoustic signal obtainment from the imaging parameters instructed by the user and transmits the imaging command to the probe 101.

### Step S207

Next, in step S207, it is determined whether or not the imaging command is appropriately transmitted. In a case where the communication interface 114 can receive an acknowledgment response from the probe 101 with respect to the imaging command transmission in step S206, it is determined that the transmission can be appropriately performed, and the flow progresses to step S208. In a case where the acknowledgment response is not received, retransmission of the imaging command is performed several times. In a case where the acknowledgment response is not received even after the retransmission is performed several times, the flow progresses to step S216.

### Step S208

In step S208, the communication interface 114 receives the ultrasonic signal data and the photoacoustic signal data transmitted from the probe unit. The received pieces of data are saved in the FIFO memory in the communication interface 114 and sequentially read out from the signal processing circuit 115.

### Step S209

Next, in step S209, classification of the types of the data and rearrangement are performed on the basis of the header information of the received data. When the retransmission of part of the data pieces after packet division is performed at the time of the wireless communication, a sequence relationship of the data may be changed in some cases. In response to this, the data pieces are rearranged on the basis of sequence numbers recorded in the header information to restore an original order. This processing is performed in the communication interface in conformity to TCP/IP protocol. With regard to the ultrasonic signal data and the photoacoustic signal data after the rearrangement, the types of the data are identified in accordance with the additional information and saved in the memory 116.

### Step S210

Next, in step S210, the signal processing unit 115 checks whether or not some data is missing. In the case of the ultrasonic signal, it is checked whether or not all pieces of the ultrasonic signal data corresponding to the single ultrasonic wave transmission beam exist. In the case of the photoacoustic signal, it is checked whether or not all pieces of the photoacoustic signal data corresponding to the single pulsed light irradiation exist. In a case where all the data pieces exist, the flow proceeds to step S211. In a case where all some data is missing even when a certain period of time elapses, the flow proceeds to step S217.

### Step S211

Next, in step S211, the signal processing unit performs the signal processing in accordance with the type of the data with respect to the ultrasonic signal data and the photoacoustic signal data to save the result in the memory 116.

### Step S212

Next, in step S212, the image generation unit 117 reads out the data from the memory 116 and performs the image generation processing in accordance with the type of the data to save the result in the memory 118.

### Step S213

Next, in step S213, the control unit 119 sequentially reads out the image data saved in the memory 118 to be displayed on the display of the user interface 120.

### Step S214

Next, in step S214, the control unit 119 determines whether or not the imaging is continued on the basis of the instruction from the user. In a case where the imaging is continued, the flow proceeds to step S206. In a case where the user issues an imaging end instruction via the user interface 120, it is determined that the imaging is not continued, and the processing is ended. In a case where the user changes the imaging parameters via the user interface, it is determined that the imaging is continued, and the flow proceeds to step S205. Then, the imaging parameters changed in step S205 are obtained, and the processing is continued. In a case where the user does not particularly issue an instruction, it is determined that the imaging is continued, and the flow proceeds to step S205. Then, in step S205, the processing is continued by using the same imaging parameters as those so far.

In step S215, the control unit 119 displays an error message indicating that the communication with the probe is not established on the display of the user interface 120, and the processing is ended.

In step S216, the control unit 119 displays an error message indicating that the imaging command is not communicated on the display of the user interface 120, and the processing is ended.

In step S217, the control unit 119 displays a warning message indicating that reception data is missing on the user interface 120. The flow then proceeds to step S214, and the processing is continued. It should be noted that the warning message can be turned off depending on a setting of the user to continue the processing in a state in which a part of the image is missing. In this case, missing data is complemented, and the flow proceeds to step S211.

### Operation flow of the ultrasonic probe

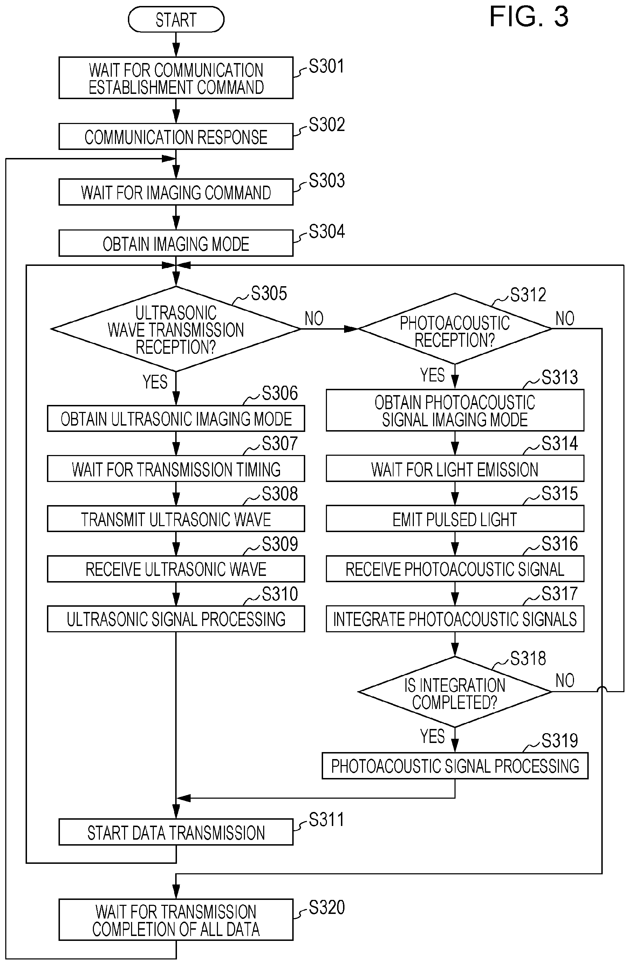

Next, an operation flow performed in the probe unit 101 will be described. Fig. 3 is a flow chart illustrating operations of the respective units of the probe unit 101.

### Step S301

After the power supply is turned on, in S301, the probe unit 101 enters a waiting state for a communication establishing command from the main body part. When the communication establishing command from the main body part 102 is received via the communication interface 110, the flow proceeds to step S302. Modules other than the communication interface 110 are put into a sleep state during the waiting state, and processing for suppressing exhaustion of the battery 113 may be performed. The communication interface 110 puts the synchronization control unit 112 from the sleep state into a normal state in a stage where the communication establishing command is received.

### Step S302

In step S302, the synchronization control unit 112 transmits the response signal to the main body part 102 via the communication interface and performs pairing of the probe unit 101 and the main body part 102. The probe ID, a corresponding communication standard, and the other information unique to the probe unit are transmitted to the main body part 102.

Specifically, the information unique to the probe unit includes information related to the light source, information related to the acoustic wave receiving unit, and other information related to the probe. The information related to the light source includes a type of the wavelength of the light source 111, the maximum frequency at which the light can be emitted, a life of the light source, and the light amount of the light source. The information related to the acoustic wave receiving unit includes the number of elements of the acoustic wave receiving unit 104, the bandwidth, and the center frequency. The other information related to the probe includes information such as the number of bits of the A/D converter, the remaining amount of the battery 113, and the communication speed of the communication interface 110.

### Step S303

Next, in step S303, the probe unit 101 enters the imaging command waiting state from the main body part 102. When the imaging command from the main body part 102 is received via the communication interface 110, the flow proceeds to step S304. The modules other than the communication interface 110 are put into the sleep state during the waiting state, and the exhaustion of the battery 113 may be suppressed. In this case, the communication interface 110 puts the synchronization control unit 112 from the sleep state into the normal state in a stage where the imaging command is received.

### Step S304

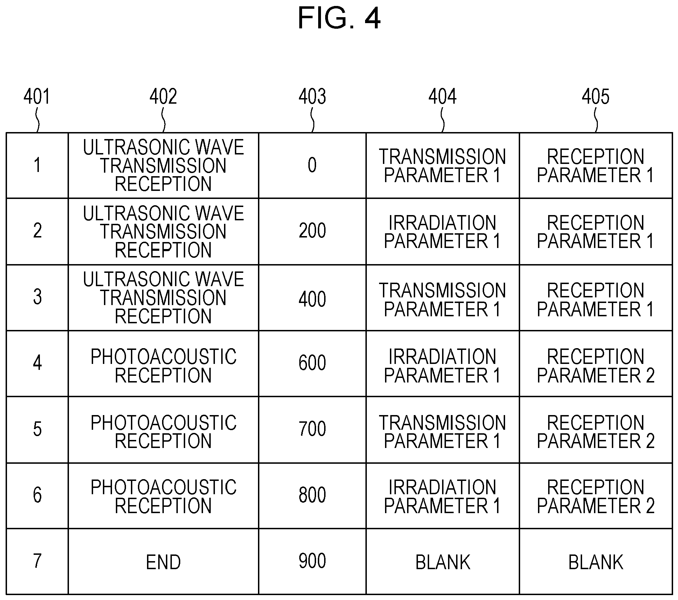

Next, in step S304, the synchronization control unit 112 analyzes packets of the received imaging command to find out the order and the number of times to perform the transmission and reception of the ultrasonic wave and the photoacoustic wave reception and a time interval to perform task scheduling. The series of tasks is saved in a task memory in the synchronization control unit 112. Herein, as a simple example, Fig. 4 illustrates contents of the task memory in a case where the ultrasonic wave transmission and reception are performed three times at an interval of 200 us, and thereafter the photoacoustic signal reception is performed three times at an interval of 100 us. Fig. 4 illustrates a task ID number 401 and a task type 402. The task type 402 includes three types of ultrasonic wave transmission and reception, photoacoustic wave reception, and end according to the present exemplary embodiment. Fig. 4 illustrates a task execution time 403 represented in units of microsecond (us). An area 404 is an area where parameters unique to the ultrasonic wave transmission are saved at the time of the ultrasonic wave transmission and reception, and parameters unique to the light irradiation are saved at the time of the photoacoustic reception. A number of the transmission parameter is saved here, and details of the individual parameters refer to other areas. An area 405 is an area where parameters unique to the ultrasonic wave reception are saved at the time of the ultrasonic wave transmission and reception, and parameters of the photoacoustic signal reception are saved at the time of the photoacoustic reception. The number of the reception parameter is saved here, and details of the individual parameters refer to other areas. With this configuration, an advantage is attained that a flexible response can be made even in a case where the number of types of the parameters is increased.

### Step S305

Next, in step S305, the synchronization control unit 112 reads out the initial task memory and determines whether or not the task type 402 is the ultrasonic wave transmission and reception. In a case where the type is the ultrasonic wave transmission and reception, the flow proceeds to step S306. In a case where the task is not the ultrasonic wave transmission and reception, the flow proceeds to step S312.

### Step S306

In step S306, the synchronization control unit 112 reads out the transmission parameter area 404 of the task memory and sets the operation of the transmission control unit 108. The synchronization control unit 112 also reads out the reception parameter area 405 and sets the operations of the amplification unit 106, the A/D conversion unit 107, and the data processing circuit 109. The transmission parameters are related to the formation of the ultrasonic wave beam to be transmitted to the subject 103 and include information such as a number of the acoustic wave receiving unit 104 to be driven, a driving voltage of the acoustic wave receiving unit 104, a driving time width, and a driving time difference between the respective acoustic wave receiving units 104. On the other hand, the reception parameters are related to the ultrasonic signal received from the subject 103 and include information such as the gain of the amplification unit 106, a TGC table, and a sampling frequency of the A/D conversion unit 107. In a case where the phasing addition, the noise removal, or the data compression is performed in the data processing circuit 109, the information includes the presence or absence of multi-stage focus at the time of the reception, a type of a digital filter, or a data compression rate.

### Step S307

Next, in step S307, the synchronization control unit 112 reads out the time area 403 of the task memory and waits until the task execution time. When the task execution time has arrived, the flow proceeds to step S308. According to the present exemplary embodiment, since the execution time of the first task is 0 us, the flow immediately proceeds to step S308.

### Step S308

Next, in step S308, the synchronization control unit 112 issues an instruction to the transmission reception switching unit 105 and connects the transmission control unit 108 and the acoustic wave receiving unit 104 to each other. Then, an instruction is issued to the transmission control unit 108 to start the ultrasonic wave transmission to the subject 103. The transmission control unit drives the acoustic wave receiving unit 104 on the basis of the transmission parameters set in step S306 and generates ultrasonic wave pulse. After the generation of the ultrasonic wave pulse, the flow proceeds to step S309. A part of the ultrasonic wave transmitted to the subject is reflected by internal tissues to be converted into the ultrasonic signal by the acoustic wave receiving unit 104.

### Step S309

Next, in step S309, the synchronization control unit 112 issues an instruction to the transmission reception switching unit 105 and connects the amplification unit 106 and the acoustic wave receiving unit 104 to each other. Then, an instruction is issued to the amplification unit 106 and the A/D conversion unit 107 to start the ultrasonic wave reception from the subject 103. The amplification unit 106 and the A/D conversion unit 107 respectively perform the amplification and the A/D conversion with respect to the ultrasonic signal on the basis of the reception parameters set in step S306. The data is saved in the memory of the data processing unit 109 after these processes.

### Step S310

Next, in step S310, the synchronization processing unit 112 issues an instruction to the data processing unit 109 and performs the signal processing on the ultrasonic signal. Specifically, the signal processing, the data integration, the compression processing, and addition of the additional information for the data identification depending on the feature of the acoustic wave receiving unit are performed. The data after the conversion is saved in the memory in the communication interface 110.

### Step S311

Next, in step S311, the synchronization control unit 112 issues an instruction to the communication interface 110 to start the data communication, and the flow returns to step S305. When the flow returns to step S305, the task memory is popped, and the initial task is deleted. In step S305, the next task is executed. The communication interface 110 performs packetization of the data saved in the memory and starts the transmission to the main body part 102. At this time, the communication interface 110 temporarily stops the communication when a communication stop signal is ON and resumes the communication when the communication stop signal turns to OFF.

### Step S312

On the other hand, in step S312, the synchronization control unit 112 reads out the initial task memory and determines whether or not the task type 402 is the photoacoustic reception. In a case where the task type is the photoacoustic reception, the flow proceeds to step S313. In a case where the task type is not the photoacoustic reception, the state corresponds to the end. It is determined that the imaging instructed from the main body part 102 is all completed, and the flow proceeds to step S320.

### Step S313

In step S313, the synchronization control unit 112 reads out the irradiation parameters area 404 of the task memory and sets the operation of the light source 111. The synchronization control unit 112 also reads out the reception parameter area 405 and sets the operations of the amplification unit 106, the A/D conversion unit 107, and the data processing circuit 109. The irradiation parameters are related to the pulsed light with which the subject 103 is irradiated and include information such as the driving power of the semiconductor laser, the wavelength, and the pulse width. On the other hand, the reception parameters are related to the photoacoustic signal received from the subject 103 and include information such as the gain of the amplification unit 106, the TGC table, and the sampling frequency of the A/D conversion unit 107. In a case where the data processing circuit 109 performs the noise removal processing of the data and the data compression, the information includes the type of the digital filter, the compression rate, and the like.

### Step S314

Next, in step S314, the synchronization control unit 112 reads out the time area 403 of the task memory and waits until the task execution time. When the task execution time has arrived, the flow proceeds to step S315. According to the present exemplary embodiment, since the execution time of the first task of the photoacoustic reception is 1100 us, when the time reaches the time 1100 us, the flow proceeds to step S315.

### Step S315

Next, in step S315, the synchronization control unit 113 transmits an instruction to the communication interface 110 to set the communication stop signal as ON. In response to this, the communication interface 110 interrupts the data communication to the main body part.

Next, in step S315, the synchronization control unit 112 issues an instruction to the transmission reception switching unit 105 and the amplification unit 106 and the acoustic wave receiving unit 104 to each other. Then, an instruction is issued to the light source 111 to start the light irradiation with respect to the subject 103. The light source 111 drives the semiconductor laser device on the basis of the irradiation parameters set in step S306 and emits the pulsed light at a time width between approximately 10 ns to approximately 200 ns. After the emission of the pulsed light, the flow proceeds to step S316. The pulsed light with which the subject is irradiated is partially absorbed by the internal tissues of the subject, and the photoacoustic wave is generated. The photoacoustic wave is converted into the photoacoustic signal by the acoustic wave receiving unit 104.

### Step S316

Next, in step S316, the synchronization control unit 112 receives the photoacoustic signal, and the amplification and the A/D conversion are performed in the amplification unit 106 and the A/D conversion unit 107.

### Step S317

Next, in step S317, the integration of the photoacoustic signal data is performed. The integration data up to the previous light irradiation is read out from the memory in the data processing circuit 109, and the newly obtained photoacoustic signal is added to be rewritten back to the memory in the data processing circuit. The memory is set as a dual port memory, and the processing can be accelerated when the read and the write are processed at the same time. In addition, reduction in the memory capacity may be realized by changing the bit width of the used memory area in accordance with the number of times to perform the integration.

### Step S318

Next, in step S318, the synchronization control unit 112 determines whether or not the integration is to be ended. According to the present exemplary embodiment, in a case where the integration is performed the previously determined number of times or the imaging is ended, the integration is ended. For example, when the three pieces of photoacoustic signal data corresponding to the light irradiation performed three times are integrated to generate the single integrated photoacoustic signal data, it is determined that the integration is ended. In a case where it is determined that the integration is ended, the flow proceeds to step S319, and the generated data is transmitted to the main body part 102. In a case where it is determined that the integration is not ended, the flow returns to step S305.

### Step S319

Next, in step S319, the synchronization processing unit 112 issues an instruction to the data processing unit 109 and performs the signal processing on the integrated photoacoustic signal. Specifically, the signal processing, the noise removal processing, the compression processing, and the addition of the additional information for the data identification depending on the feature of the acoustic wave receiving unit are performed. The data after the conversion is saved in the memory in the communication interface 110. Next, the flow progresses to step S311, the photoacoustic signal is transmitted to the main body part 102.

### Step S320

In step S320, the synchronization control unit 112 stands by until the transmission of all the pieces of data to the main body part 102 is ended. The determination as to whether or not all the data has been transmitted to the main body part 102 can be realized by monitoring a state of the communication interface 110. When all the data has been transmitted to the main body part, the flow proceeds to step S303.

### Time chart

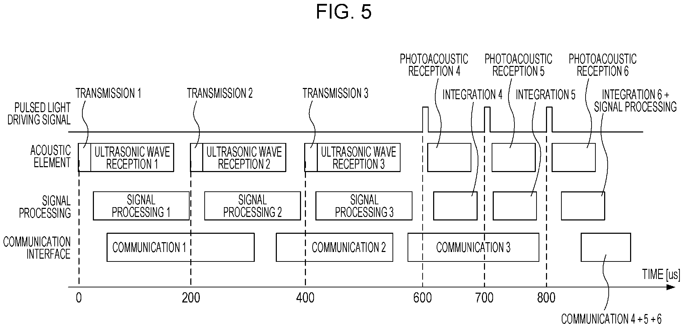

Fig. 5 illustrates an example of a time chart in a case where the ultrasonic wave transmission and reception and the photoacoustic reception are respectively performed three times. Atop part of Fig. 5 illustrates a timing of a signal (pulsed light driving signal) for emitting the pulsed light from the light source 111. The horizontal axis represents the time. A second part from the top illustrates a timing for the ultrasonic wave and photo acoustic wave reception by the acoustic wave receiving unit 104. A third part from the top illustrates a timing for the data processing circuit 109 to perform the signal processing. A fourth part from the top illustrates a timing for the communication interface 110 to communicate the ultrasonic signal data and the photoacoustic signal data with respect to the main body part 102. A fifth part from the top illustrates a timing for the communication control signal of the synchronization control unit 112. All the horizontal axes in Fig. 5 represent the time. In addition, Transmission 1 is equivalent to the ultrasonic wave transmission corresponding to the task number 1 in Fig. 4, and Ultrasonic wave reception 1 is equivalent to the ultrasonic wave reception corresponding to the task number 1. The numbers in Fig. 5 also correspond to the task numbers in Fig. 4 with respect to Transmission 2 to Transmission 3, Ultrasonic wave reception 2 to Ultrasonic wave reception 3, Photoacoustic reception 4 to Photoacoustic reception 6, Signal processing 1 to Signal processing 6, Communication 1 to Communication 6. With reference to Fig. 5, the laser irradiation and the photoacoustic signal reception are started in an interval of 100 us from the time 600. With regard to the photoacoustic signal data obtained in Photoacoustic reception 4 and Photoacoustic reception 5, the communication is not performed since it is determined in step S318 that the integration is not ended, and the integration result is saved in the memory in the data processing circuit 109. On the other hand, it is determined in step S318 that the integration is ended since three data pieces are obtained in a stage where the photoacoustic reception 6 obtains the photoacoustic signal data. Then, the data pieces after the signal processing is ended are sequentially transmitted to the main body part 102. When the photoacoustic signals are integrated in the above-described manner, the noise is reduced, and also an advantage is attained that the communication data amount is decreased. As illustrated in the situation of the communication interface from the time 600 us to the time 800 us in Fig. 5, the time freed by the integration can be used for the communication of the ultrasonic signal, which contributes the improvement in the frame rates of the ultrasonic wave and the photoacoustic signal.

When the sound speed in the subject 103 is set as 1500 m/s and the observation depth of the subject 103 is set as 100 mm, the ultrasonic wave transmission and reception take approximately 134 us, and the photoacoustic wave reception takes 67 us. With regard to the signal processing, the data after the A/D conversion can be sequentially processed, and the processing time is substantially equal to the reception time and does not largely fluctuate. On the other hand, the communication with the main body largely fluctuates depending on a situation of a location where the photoacoustic apparatus is placed at that time and a communication situation with a surrounding device. For example, in a case where the main body part 102 is away from the probe unit 101, a case where an obstacle that shields the radio wave exists in the surrounding, a case where radio interference with the surrounding device exists, or the like, the communication error or the retransmission occurs, and the communication speed tends to be largely decreased. For this reason, the time spent for the communication dynamically changes, and the next task is to be stated while the previous data is being communicated in some cases. For example, the time for Communication 2 may be longer than the time for Communication 1, and Transmission 3 may be started before Communication 2 is ended. For this reason, the communication interface 110 may be provided with a buffer memory having a sufficient capacity.

The number of elements of the acoustic wave receiving unit 104 is set as 128 channels. The bit count of the A/D conversion unit 107 is set as 12 bits, and the sampling frequency of the A/D converter is set as 40 MHz. At this time, the raw data amount of the ultrasonic signal per ultrasonic wave transmission beam becomes approximately 8.3 Mbit, and the raw data amount of the photoacoustic signal per photoacoustic wave reception becomes approximately 4.1 Mbit. With regard to the ultrasonic signal, 12-bit data pieces having 128 channels are subjected to the phasing addition by the data processing circuit 109 to be integrated into the single 19-bit data, so that the data amount of the single ultrasonic wave transmission and reception is compressed to 0.101 Mbit. When an effective speed in Communication 1 is set as 500 Mbps, Communication 1 takes 202 us. When an effective speed in Communication 2 is set as 300 Mbps, Communication 2 takes 336 us. When an effective speed in Communication 3 and subsequent communication is set as 500 Mbps, Communication 3 takes 202 us.

On the other hand, the photoacoustic signals for the three occasions are integrated and thereafter divided by the number of times to perform the integration into the single 12-bit data which has 4.1 Mbit. This is subjected to the phasing addition by 128 channels and integrated into the 19-bit data which is compressed to 0.05 Mbit. When the effective speed in Communication 4 + Communication 5 + Communication 6 is set as 500 Mbps, Communication 4 + Communication 5 + Communication 6 take 101 us.

As described above, according to the exemplary embodiment of the present invention, when the probe obtains the photoacoustic data multiple times to be subjected to the integration and thereafter transmitted to the main body part, it is possible to realize both the noise reduction and the decrease in the communication data amount. With this configuration, the frame rates of the ultrasonic signal and the photoacoustic signal can be improved.

It should be noted that, according to the present exemplary embodiment, the descriptions have been made by using the example in which both the ultrasonic wave transmission and reception and the photoacoustic reception are executed. However, even in an apparatus that has only the photoacoustic reception function without the ultrasonic wave transmission and reception function, when the light irradiation is performed multiple times and the obtained photoacoustic signals are integrated, it is possible to attain a similar communication amount reduction effect. With this configuration, the cycle of the light irradiation can be further shortened, and the number of times to perform the integration is increased, so that the noise reduction can be realized.

In addition, according to the present exemplary embodiment, the example has been illustrated in which the integration and the division of the photoacoustic signal data are performed inside the probe unit 101 to perform the calculation of the addition averaging, but only the integration may be performed in the probe unit 101, and the division may be performed in the main body part 102. As compared with a case where both the integration and the division are performed, the probe unit 101 does not need to include the division circuit at the expense of the slightly increased communication data amount, which leads to the miniaturization and the decrease in the power consumption of the probe unit.

In addition, according to the present exemplary embodiment, the descriptions have been given by using the example in which the number of times to perform the integration is three. However, a configuration may also be adopted in which the number of times to perform the integration is limited to a power of 2, and the division circuit is replaced with a shift circuit to reduce the circuit scale of the probe unit to realize the miniaturization and the decrease in the power consumption of the probe unit.

Moreover, according to the present exemplary embodiment, the descriptions have been given by using the example in which the ultrasonic wave transmission and reception are executed three times, and thereafter the photoacoustic reception is executed three times, but the number of times to perform the ultrasonic wave transmission and reception, and the number of times to perform the photoacoustic reception and the order thereof are not limited to the above and can be freely changed by the instruction of the user in Fig. 4. For example, the ultrasonic signal transmission and reception and the photoacoustic reception may be alternately performed. The number of times to perform the photoacoustic reception may also be temporarily increased to inspect a deeper part inside the subject. In addition, a change for the number of times to perform the integration may be performed by a switch, a dial, or the like attached to the probe.

Furthermore, according to the present exemplary embodiment, the descriptions have been given by using the example in which the number of times to perform the integration is fixed to three, but the number of times to perform the integration is not limited to three. For the noise reduction, the number of times to perform the integration can also be increased. For example, when the number of times to perform the integration is set as 100, the random noise ideally becomes 1/10, and an image of a light absorption section at a deeper location inside the subject can be obtained at the expense of the decrease in the frame rate. In this manner, in a case where the number of times to perform the integration is increased, the effect of the decrease in the communication data amount when the probe unit performs the integration becomes more conspicuous.

On the other hand, when the number of times to perform the integration is increased too much, the subject may move during the integration, and there is a fear that the photoacoustic image is blurred. While the number of times to perform the integration is increased, the frequency of the light source may also be increased at the same time, and the influence of the movement of the subject may be suppressed. In addition, a configuration may be adopted in which a sensor configured to detect the movement is included in the probe unit, the integration is performed when a relative positional relationship between the probe unit and the subject is not moved, and the integration is not performed when the movement exists. Moreover, the movement detection may be performed depending on a change in the ultrasonic signal data.

In addition, the example has been illustrated in which the number of times to perform the integration is fixed irrespective of the communication state according to the present exemplary embodiment, but the communication speed in a case where a wireless configuration is used momently changes depending on a situation of a surrounding electromagnetic environment. For this reason, the number of times to perform the integration may be changed in accordance with the communication speed. That is, the communication speed is estimated from the data remaining amount in the FIFO memory in the communication interface. When the communication speed is slow, the number of times to perform the integration may be increased to decrease the communication data amount. On the other hand, when the communication speed is fast, the number of times to perform the integration may be decreased to increase the frame rate of the photoacoustic image.

Similarly, the frequency of the light irradiation may be changed in accordance with the communication speed. That is, the communication speed is estimated from the data remaining amount in the FIFO memory in the communication interface. When the communication speed is slow, the frequency of the light irradiation may be decreased to decrease the communication data amount. On the other hand, when the communication speed is fast, the frequency of the light irradiation may be increased to increase the number of times to perform the integration to realize the noise reduction.

With the ultrasonic probe according to the exemplary embodiment of the present invention, it is possible to provide the ultrasonic probe that can reduce the communication data amount at the time of the communication of the signals obtained when the subject is irradiated with the light multiple times and the photoacoustic apparatus including the ultrasonic probe.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

1. An ultrasonic probe (101) comprising:
light irradiation means (111) configured to irradiate a subject (103) with light;
acoustic wave receiving means (104) configured to receive an acoustic wave generated when the subject is (103) irradiated with the light and convert the acoustic wave into an electric signal;
analog-to-digital conversion means (107) configured to convert the electric signal into a digital signal;
integration means (109) configured to integrate a plurality of the digital signals obtained when the subject is irradiated with the light multiple times and output an integrated signal; and
communication means (110) configured to perform a communication of the integrated signal.

2. The ultrasonic probe according to Claim 1, wherein the communication means is configured to perform a wireless communication of the integrated signal.

3. The ultrasonic probe according to Claim 1 or Claim 2, wherein the integration means (109) is configured to be able to perform division processing for dividing the integrated signal.

4. The ultrasonic probe according to Claim 1 or Claim 2, wherein the integration means (109) is configured to be able to change the number of times to perform the integration of the digital signals.

5. The ultrasonic probe according to Claim 1 or Claim 2, wherein the acoustic wave receiving means (104) is configured to be able to perform ultrasonic wave transmission.

6. The ultrasonic probe according to Claim 1 or Claim 2, wherein the light irradiation means (111) includes a light source constituted by including a plurality of semiconductor light emitting elements.

7. A photoacoustic apparatus comprising:
the ultrasonic probe (101) according to any one of Claims 1 to 6; and
signal processing means (102) configured to obtain information related to the subject from the communication means (110).

8. The photoacoustic apparatus according to Claim 7, further comprising storage means (116) that saves the digital signals,
wherein the light emitted from the light irradiation means is pulsed light, and
wherein, in a case where a data amount saved in the memory means (110) is higher than or equal to a predetermined value, control is performed to decrease a repetition frequency of the pulsed light emitted from the light irradiation means.
